# EUROPÄISCHE PATENTSCHRIFT

(11) **EP 2 613 793 B1**
(45) Veröffentlichungstag und Bekanntmachung des Hinweises auf die Patenterteilung: **18.11.2015**
(21) Anmeldenummer: 11758157.9
(22) Anmeldetag: 01.09.2011
(51) Int. Cl.: A61P 11/00, A61P 11/02, A61K 31/74, A61K 33/14, A61K 36/61, A61K 36/53, A61K 36/534, A61K 9/00, A61K 9/08, A61K 47/38, A61K 31/045, A61K 31/164

(54) **NASENSPRAY**
NASAL SPRAY
SPRAY NASAL

(30) Priorität: 07.09.2010 DE 202010012255 U
(43) Veröffentlichungstag der Anmeldung: 17.07.2013
(73) Patentinhaber: Krewel Meuselbach GmbH, 53783 Eitorf (DE)
(72) Erfinder: SCHIERSTEDT, Detlef, 53757 St. Augustin (DE)
(74) Vertreter: Von Kreisler Selting Werner - Partnerschaft von Patentanwälten und Rechtsanwälten mbB
(86) Internationale Anmeldenummer: PCT/EP2011/065132
(87) Internationale Veröffentlichungsnummer: WO 2012/031979

(56) Entgegenhaltungen:
- WO-A1-2006/134406
- DE-A1-102009 005 865
- DE-U1-202008 016 832
- US-A1- 2005 232 868
- KEHRL W ET AL: "Dexpanthenol-Nasenspray als wirksames Therapieprinzip zur Behandlung der Rhinitis sicca anterior // Dexpanthenol nasal spray as an effective therapeutic principle for treatment of rhinitis sicca anterior", LARYNGO-RHINO-OTOLOGIE, THIEME, STUTTGART, DE, DE, Bd. 77, Nr. 9, 1. September 1998 (1998-09-01), Seiten 506-512, XP009144282, ISSN: 0935-8943

## Beschreibung

Die Erfindung betrifft ein Sympathomimetika-freies Medizinprodukt, welches sich vorteilhaft auf die Nasenschleimhäute bei vulgärem Schnupfen, Heuschnupfen oder anderen Rhinitiden, trockener Nase und/oder Sympathomimetika Abhängigkeiten auswirkt, in Form eines Nasensprays, einer Spülung oder Nasentropfen.

Bei vulgärem Schnupfen (Rhinitis catarrhalis), Heuschnupfen, anderer Rhinitiden oder trockener Nase kommt es zur Anschwellung der Nasenschleimhäute. Es ist bisher üblich, zur Behandlung Sympathomimetika lokal in Form von Nasentropfen oder Nasensprays anzuwenden, um eine Schleimhautabschwellung zu erreichen. Sympathomimetika besitzen jedoch zahlreiche unerwünschte Nebenwirkungen und können infolge einer Resorption zu Herzklopfen und Atemstörungen führen. Darüber hinaus verursachen sie die Austrocknung der Nasenschleimhaut und bei längerer Anwendung kommt es zu einer dauerhaften Nasenschleimhaut-Epithel-Schädigung. Eine solche Sympathomimetika Abhängigkeit ist als Rhinopathia medicamentosa bekannt (Apotheker-Journal 12, 30 - 34 (1985) Otto Hoffmanns Verlag, München).

EP 0 216 917 B1 betrifft ein therapeutisches Präparat zur nasalen Verabreichung, das unter anderem ein Andickungsmittel enthält. Als Andickungsmittel wird beispielsweise Methylcellulose genannt. US 5,843,881 A betrifft eine Sprayzusammensetzung. Die Zusammensetzung enthält insbesondere Alkohol, ein Polymer und einen Alkoholmaskierenden Parfumzusatz. Die Zusammensetzungen werden auf die Haut, das Haar oder die Schleimhaut aufgetragen.

US 2005/232868 A1 beschreibt ein Verfahren zur Prävention und Behandlung von viralen Infektionen des oberen Atemwegtraktes durch Verabreichung zum Bespiel eines Nasensprays, das Polyethylenglycol, Menthol, Pfefferminzöl, Campher, Hydroxymethylcellulose, Kochsalz etc. enthalten kann, siehe insbesondere Tabelle 1 und 2 auf Seite 7.

Kehrl W. et al.: "Dexpanthenol-Nasenspray als wirksames Therapieprinzip zur Behandlung der Rhinitis sicca anterior // Dexpanthenol nasal spray as an effective therapeutic principle for treatment of rhinitis sicca anterior", Laryngo-Rhino-Otologie, Thieme, Stuttgart, DE, DE, Bd. 77, Nr. 9, 1. September 1998 (1998-09-01), Seiten 506-512, XP009144282, ISSN: 0935-8943 beschreibt eine Untersuchung der Effizienz der Applikation von Dexpanthenol in physiologischer Kochsalzlösung (Nasicur) als Nasenspray, welche in einer effektiven medizinischen Behandlung der Rhinitis sicca anterior resultierte, die sich als effektiver als herkömmliche Medikamente erwies.

DE 10 2009 005 865 A1 betrifft eine Dosiereinheit, insbespndere in Schlauch-, Beutel- oder Tütenform, wobei die Dosiereinheit eine Umverpackung mit einer hierin eingebrachten und/oder hiervon eingeschlossenen, wässrig basierten, flüssigen, gefrierfähigen Zusammensetzung, insbesondere pharmazeutischen Zusammensetzungen, umfasst, wobei die Zusammensetzung im festen, gefrorenen Zustand zur prophylaktischen oder kurativen topischen (lokalen) Behandlung von entzündlichen Erkrankungen des Mund- und Rachenraums, insbesondere mittels Lutschen und/oder Kauen, verabreicht wird und eine wirksame, insbesondere therapeutisch und/oder pharmazeutisch wirksame Menge mindestens eines desinfizierenden und/oder entzündungshemmenden und/oder schmerzlindernden Wirkstoffs, insbesondere pharmazeutischen Wirkstoffs, enthält.

DE 2008 016 832 U1 betrifft eine Zusammensetzung, insbesondere pharmazeutische Zubereitung in Form einer flüssigen Dosierung, insbesondere zur vorzugsweise topischen (lokalen) prophylaktischen oder therapeutischen (kurativen) Behandlung von insbesondere entzündlichen Erkrankungen des Mund- und Hals-/-Rachenraums, wobei die Zusammensetzung in Kombination und in jeweils wirksamen, insbesondere pharmazeutisch wirksamen Mengen
(a) mindestens eine Schleimdroge,
(b) Dexpanthenol (Panthenol, Pantothenol) und/oder Pantothensäure oder deren Derivate, insbesondere deren Ester oder Salze, und
(c) Holunder, insbesondere Holunderblüten, enthält.

WO 2006/134406 A1 beschreibt stabile pharmazeutische Gele zur topischen (lokalen) Verabreichung von Diclofenac durch die Haut, zum Beispiel bei Weichgewebeerkrankungen, die als Zusatzstoffe Menthol und Methocel E4 MP enthalten.

In den letzten Jahren haben sich Sympathomimetika-freie Nasensprays auf Basis von Meersalz und/oder Kochsalz durchgesetzt. Diese haben keinen akuten pharmakologischen Effekt (Medizinprodukt) auf die Nasenschleimhaut, sind aber in der Lage durch die Befeuchtung und Reinigung der Schleimhaut, insbesondere nach mehrmaliger Anwendung, die Schleimhäute zu beruhigen und so eine Linderung der Beschwerden hervorzurufen.

Da die Verweilzeit der Lösung nur kurz ist, muss die Lösung sehr häufig appliziert werden und die Linderung reicht bei stärkeren Beschwerden oft nicht aus.

Die Aufgabe der vorliegenden Erfindung liegt daher in der Bereitstellung eines Medizinpröduktes, welches im Vergleich zu bekannten Produkten stärker zur Beruhigung der Nasenschleimhaut führt. Dabei soll es jedoch keine Abhängigkeiten bewirken, wie dies bei Sympathomimetika enthaltenden Produkten der Fall ist.

Überraschenderweise wurde gefunden, dass Menthol, Minzöl und Campher zusammen mit einem Feuchthaltemittel sich befreiender auf die Nasenschleimhäute auswirken, als die üblichen Kochsalz und/oder Meersalz enthaltenden Sprays. Eine besondere Ausführungsform enthält zusätzlich noch Pantothenol, insbesondere Dexpantothenol.

Eine erste Ausführungsform der Erfindung umfasst daher ein Sympathomimetika-freies Medizinprodukt zur Beruhigung der Nasenschleimhaut gemäß Anspruch 1. Dieses ist dadurch gekennzeichnet ist, dass es Menthol, Minzöl **und Campher sowie** Pantothenol, insbesondere Dexpantothenol, sowie wenigstens ein Feuchthaltemittel, welches wasserlösliche oder in Wasser dispergierbare natürliche oder synthetische Polymere, die in wässrigen Systemen Gele oder viskose Lösungen bilden, mehrwertige Alkohole, Mono-, Di- und/oder Polysaccharide umfasst, umfasst. Polymere werden bevorzugt verwendet. Geeignete Polymere sind beispielsweise Kollagenderivate (tierische und pflanzliche), Polyalkylenglykole, insbesondere Polyethylenglykole, Polyglycerine, Alginate, Carageen, Pektine, Tragant, Gumen, insbesondere Gummi Arabicum, Cellulosederivate, insbesondere Celluloseether und/oder Celluloseester, Polyvinylalkohole, Polyvinylpyrrolidone und Derivaten hiervon und/oder Dextran.

Besonders bevorzugte Cellulosederivate im Sinne der vorliegenden Erfindung umfassen Hydroxyethylcellulose und/oder Methylhydroxypropylcellulose.

Das erfindungsgemäße Medizinprodukt weist Campher auf.

Die Kombination aus Feuchthaltmittel und Pantothenol zusammen mit Menthol und Minzöl und Campher bewirkt eine Befeuchtung der Nasenschleimhaut. Zusätzlich kommt es zu einer Beruhigung der Nasenschleimhaut. Menthol und Minzöl und Campher üben einen kühlenden Effekt auf die Nasenschleimhaut aus. In Kombination mit dem Feuchthaltemittel kommt es zu einer Beruhigung der Nasenschleimhaut. Überraschenderweise wurde gefunden, dass sich in einem erfindungsgemäßen Nasenspray diese beiden physikalischen Effekte optimal ergänzen und eine verbesserte Wirkung gegenüber Meersalz und/oder Kochsalz haltigen Nasensprays aufweisen.

Beruhigend auf die Nasenschleimhäute im Sinne der vorliegenden Erfindung bedeutet das Feuchthalten der Nasenschleimhäute. Das bei Verwendung Sympathomimetika-haltiger Nasensprays auftretende Trockenheitsgefühl tritt beim erfindungsgemäßen Nasenspray nicht auf. Darüber hinaus umfasst "Beruhigung" auch eine geringere Anschwellung oder sogar Abschwellung der gereizten Nasenschleimhaut.

Bei regelmäßigem Gebrauch stellt das erfindungsgemäße Präparat eine Alternative zu Sympathomimetika-haltigen Nasensprays dar. Hiervon können dann auch solche Personen profitieren, welche von Sympathomimetika-haltigem Nasenspray abhängig sind.

Mehrere Probanden, welche bei vulgärem Schnupfen normalerweise auf den Gebrauch Sympathomimetika-haltigen Sprays angewiesen waren, erhielten über den Zeitraum von einer Woche das erfindungsgemäße Produkt in Form eines Sprays. Zwei Probanden konnten während der Infektionsperiode von einer Woche ganz auf Sympathomimetika-haltige Sprays verzichten. Vier Probanden reduzierten bei gleichzeitiger Anwendung des erfindungsgemäßen Produkts den Gebrauch von Sympathomimetika-haltigen Sprays auf die Hälfte.

Weitere fünf Probanden mit einer Abhängigkeit von Sympathomimetikahaltigen Nasensprays hatten alternativ das erfindungsgemäße Spray verwendet. Um nachts durchatmen zu können, verwendeten die Probanden vor dem Einschlafen bisher Sympathomimetika-haltige Sprays, welche ganz oder teilweise durch das erfindungsgemäße Spray ersetzt wurden. Nach zwei Wochen konnten einer der fünf Probanden ganz auf die Anwendung Sympathomimetika-haltiger Nasensprays verzichten. Die übrigen konnten den Gebrauch der Sympathomimetikahaltigen Sprays auf etwa die Hälfte reduzieren.

Erfindungsgemäß kann das Medizinprodukt wenigstens ein Feuchthaltemittel in einer Menge von 0,1 bis 5 Gew.-%, insbesondere in einer Menge von 0,5 bis 2 Gew.-% enthalten.

Eine zu geringe Menge des wenigstens einen Feuchthaltemittels verhindert eine langandauernde Filmbildung, während eine zu große Menge des wenigstens einen Feuchthaltemittels die Viskosität der Medizinprodukte unerwünscht erhöht.

In einer weiteren Ausführungsform enthält das erfindungsgemäße Medizinprodukt Minzöl und Menthol und Campher bis zu deren Sättigungsgrenze. Bei höheren Konzentrationen ist eine homogene Phase im erfindungsgemäßen Medizinprodukt nicht sicherzustellen. Die Konzentration an Minzöl und Menthol und Campher würde somit bei jeder Anwendung stark variieren. Bei der Anwendung käme es außerdem zu einer inhomogenen Benetzung der Nasenschleimhaut nicht nur mit Minzöl, Menthol und Campher. Auch eine homogene Auftragung von Pantothenol und Feuchthaltemittel kann nicht gewährleistet werden.

Ein erfindungsgemäßes Medizinprodukt weist bevorzugt mehr als 0,002 g, insbesondere mehr als 0,003 g Minzöl und Menthol auf. Bevorzugt weist das Medizinprodukt mehr als 0,002 g Campher, insbesondere mehr als 0,003 g Campher auf. Bei geringeren Anteilen ist eine ausreichende beruhigende Wirkung auf die Nasenschleimhaut nicht gewährleistet.

Eine weitere bevorzugte Ausführungsform der vorliegenden Erfindung ist dadurch gekennzeichnet, dass das Medizinprodukt weiterhin zusätzlich Kochsalz und/oder Meersalz enthält. Anstelle oder in Kombination damit, können auch weitere tonisierende Zusätze wie physiologische Salze, Puffer oder auch ionische oder nichtionische physiologisch verträgliche Stoffe als Basis dienen. Die Verwendung von Kochsalz oder Meersalz ist im Stand der Technik weit verbreitet. Entsprechende Mittel können dabei natürliches Meerwasser mit Spurenelementen und Mineralstoffen enthalten.

Mit Hilfe von pH-Wert-Regulatoren ist es möglich, den pH-Wert der erfindungsgemäßen Medizinprodukte auf einen physiologisch verträglichen pH-Wert, möglichst nicht unter pH 6,5, einzustellen. Besonders bevorzugte pH-Wert-Regulatoren im Sinne der vorliegenden Erfindung sind Natriumhydroxid, Natriumphosphat, Natriumcitrat und andere physiologisch verträgliche Puffersysteme, beispielsweise Phosphat oder Citratpuffer. Der obengenannte Grenzwert entspricht dem für Nasenspray üblichen pH-Wert, der durch die Schleimhautverträglichkeit bestimmt wird.

Die Tonie der erfindungsgemäßen Medizinprodukte wird üblicherweise in Richtung einer leichten Hypertonie (besonders bevorzugt 400 mosmol) eingestellt. Besonders bevorzugte Tonisierungsmittel umfassen insbesondere Glukose, Sorbit, Mannit und/oder Xylit. Bei einer hypotonen Lösung (weniger 290 mosmol) besteht die Gefahr, dass die Zellen der Nasenschleimhaut geschädigt werden. Stark hypertone Lösungen werden als unangenehm empfunden.

Die galenische Zubereitung der erfindungsgemäßen Medizinprodukte kann nach den galenischen Methoden und Regeln erfolgen, wie sie für die Herstellung von wässrigen Nasentropfen allgemein üblich sind (Sucker, H. P. Fuchs und P. Speiser: Pharmazeutische Technologie, Thieme Verlag, Stuttgart (1978)). Besonders bevorzugt im Sinne der vorliegenden Erfindung werden die Medizinprodukte in Form einer wässrigen Lösung hergestellt.

In einer weiteren Ausführungsform umfasst das Medizinprodukt ferner ätherische Öle. Bevorzugt im Sinne der vorliegenden Erfindung sind Thymianöl, Eucalyptusöl und/oder Salbeiöl. Diese sorgen für einen angenehmen Geruch des Medizinproduktes.

Ein erfindungsgemäßes Sympathomimetika-freies Medizinprodukt ist bevorzugt frei von Konservierungsmitteln. Durch diese würde der abschwellende Effekt an die Nasenschleimhäute vermindert werden. Vielmehr rufen solche häufig erneut eine Reizung der Nasenschleimhäute vor und schädigen die Zilien der Nase. Erfindungsgemäß liegt das Medizinprodukt insbesondere in einer weiteren Ausführungsform in Form einer wässrigen Lösung vor. Einer solchen wässrigen Lösung können die ätherischen Öle bis zu ihrer maximalen Löslichkeit zugegeben werden.

Eine weitere Ausführungsform betrifft die Verwendung des erfindungsgemäßen Medizinprodukts. Dieses wird besonders bevorzugt bei Nasenschleimhautreizungen eingesetzt, die hervorgerufen werden durch Schnupfen oder Heuschnupfen. Ursache kann auch eine trockene Nase, hervorgerufen unter anderem durch trockene Heizungsluft sein. Auch bei Sympathomimetika-Abhängigkeit bestehende Nasenschleimhautreizungen können durch das erfindungsgemäße Medizinprodukt gelindert werden. Bevorzugt wird das Medizinprodukt gemäß der vorliegenden Erfindung in Form von Tropfen, Sprays oder Spüllösungen verwendet.

## Patentansprüche

1. Sympathomimetika-freies Medizinprodukt in Form **eines Nasensprays, einer Spülung oder Nasentropfen** zur Verwendung zur Herstellung eines Mittels zur Behandlung von Nasenschleimhautreizungen, hervorgerufen durch Schnupfen, Heuschnupfen, trockene Nase und/oder Sympathomimetika Abhängigkeit, **dadurch gekennzeichnet, dass** es Menthol, Minzöl und Campher sowie **weiterhin Pantothenol und** wenigstens ein Feuchthaltemittel, welches wasserlösliche oder in Wasser dispergierbare natürliche oder synthetische Polymere, die in wässrigen Systemen Gele oder viskose Lösungen bilden, mehrwertige Alkohole, Mono-, Di- und/oder Polysaccharide umfasst, umfasst.

2. Sympathomimetika-freies Medizinprodukt zur Verwendung nach Anspruch 1, **dadurch gekennzeichnet, dass** die Polymere ausgewählt sind aus Kollagenderivaten, Polyalkylenglykolen, insbesondere Polyethylenglykolen, Polyglycerinen, Alginaten, Carageen, Pektinen, Tragant, Gumen, insbesondere Gummi Arabicum, Cellulosederivaten, insbesondere Celluloseether und/oder Celluloseester, Polyvinylalkoholen, Polyvinylpyrrolidonen und Derivaten hiervon und/oder Dextran.

3. Sympathomimetika-freies Medizinprodukt zur Verwendung nach Anspruch 1 bis **2**, **dadurch gekennzeichnet, dass** es das Feuchthaltemittel in einer Menge von 0,1 bis 5 Gew.-% , insbesondere in einer Menge von 0,5 bis 2 Gew.-%, enthält.

4. Sympathomimetika-freies Medizinprodukt zur Verwendung nach einem der Ansprüche 1 bis **3**, **dadurch gekennzeichnet, dass** es weiterhin zusätzlich Kochsalz und/oder Meersalz enthält.

5. Sympathomimetika-freies Medizinprodukt zur Verwendung nach einem der Ansprüche 1 bis **4**, **dadurch gekennzeichnet, dass** es außerdem ätherische Öle, ausgewählt aus Thymianöl, Eukalyptusöl und/oder Salbeiöl, enthält.

6. Sympathomimetika-freies Medizinprodukt zur Verwendung nach einem der Ansprüche 1 bis **5**, **dadurch gekennzeichnet, dass** es weiterhin pH-Regulatoren und/oder Tonisierungsmittel, insbesondere Zucker, ausgewählt aus Glucose, Sorbit, Mannit und/oder Xylit enthält.

7. Sympathomimetika-freies Medizinprodukt zur Verwendung nach einem der Ansprüche 1 bis **6** in Form einer wässrigen Lösung.

8. Verwendung eines Sympathomimetika-freien Medizinproduktes nach einem der Ansprüche 1 bis **7** zur Herstellung eines Mittels zur Behandlung von Nasenschleimhautreizungen, hervorgerufen durch Schnupfen, Heuschnupfen, trockene Nase und/oder Sympathomimetika Abhängigkeit.

## Claims

1. A sympathomimetic-free medicinal product in the form of nose sprays, rinses or nose drops, for use for preparing an agent for treating irritations of the nasal mucosa caused by a cold, by hay fever, a dry nose and/or sympathomimetic dependence, **characterized by** comprising menthol, mint oil and camphor, as well as further panthenol, and at least one humectant comprising water-soluble or water-dispersible natural or synthetic polymers that form gels or viscous solutions in aqueous systems, polyhydric alcohols, mono-, di- and/or polysaccharides.

2. The sympathomimetic-free medicinal product for use according to claim 1, **characterized in that** said polymers are selected from collagen derivatives, polyalkylene glycols, especially polyethylene glycols, polyglycerols, alginates, carrageenan, pectins, tragacanth gum, gums, especially gum arabic, cellulose derivatives, especially cellulose ethers and/or cellulose esters, polyvinyl alcohols, polyvinyl pyrrolidones and derivatives thereof, and/or dextran.

3. The sympathomimetic-free medicinal product for use according to claim 1 to 2, **characterized by** containing said humectant in an amount of from 0.1 to 5% by weight, especially in an amount of from 0.5 to 2% by weight.

4. The sympathomimetic-free medicinal product for use according to any of claims 1 to 3, **characterized by** further additionally containing common salt and/or sea salt.

5. The sympathomimetic-free medicinal product for use according to any of claims 1 to 4, **characterized by** additionally containing essential oils selected from thyme oil, eucalyptus oil and/or sage oil.

6. The sympathomimetic-free medicinal product for use according to any of claims 1 to 5, **characterized by** further containing pH control agents and/or tonicizing agents, especially sugars selected from glucose, sorbitol, mannitol and/or xylitol.

7. The sympathomimetic-free medicinal product for use according to any of claims 1 to 6 in the form of an aqueous solution.

8. Use of a sympathomimetic-free medicinal product according to any of claims 1 to 7 for preparing an agent for treating irritations of the nasal mucosa caused by a cold, by hay fever, a dry nose and/or sympathomimetic dependence.

## Revendications

1. Produit médical exempt de sympathomimétiques sous forme de vaporisateur nasal, de rince-nez ou de gouttes pour le nez conçu pour être utilisé pour la production d'un agent pour le traitement d'irritations des muqueuses nasales provoquées par un rhume, un rhume des foins, un nez sec et/ou une dépendance de sympathomimétiques, **caractérisé en ce qu'**il comprend du menthol, de l'huile de menthe et du camphre ainsi que, en outre, du panthénol et au moins un humectant comprenant des polymères naturels ou synthétiques, solubles dans l'eau ou dispersibles dans l'eau, formant des gels ou des solutions visqueuses dans des systèmes aqueux, des alcools polyhydriques, des monosaccharides, des disaccharides et/ou des polysaccharides.

2. Produit médical exempt de sympathomimétiques conçu pour être utilisé selon la revendication 1, **caractérisé en ce que** les polymères sont choisis parmi les dérivés de collagène, les polyalkylène glycols, notamment les polyéthylène glycols, les polyglycérols, les alginates, la carraghénine, les pectines, la gomme adragante, des gommes, notamment la gomme arabique, les dérivés de cellulose, notamment les éthers de cellulose et/ou les esters de cellulose, les alcools polyvinyliques, les polyvinylpyrrolidones et les dérivés de celles-ci, et/ou le dextrane.

3. Produit médical exempt de sympathomimétiques conçu pour être utilisé selon la revendication 1 à 2, **caractérisé en ce qu'**il contient ledit humectant à raison de 0,1 à 5 % en poids, notamment à raison de 0,5 à 2 % en poids.

4. Produit médical exempt de sympathomimétiques conçu pour être utilisé selon l'une quelconque des revendications 1 à 3, **caractérisé en ce qu'**il contient en outre du sel ordinaire et/ou du sel marin.

5. Produit médical exempt de sympathomimétiques conçu pour être utilisé selon l'une quelconque des revendications 1 à 4, **caractérisé en ce qu'**il contient en outre des huiles essentielles choisies parmi l'huile de thym, l'huile d'eucalyptus et/ou l'huile de sauge.

6. Produit médical exempt de sympathomimétiques conçu pour être utilisé selon l'une quelconque des revendications 1 à 5, **caractérisé en ce qu'**il contient en outre des agents de contrôle du pH et/ou des agents fortifiants, notamment des sucres choisis parmi le glucose, le sorbitol, le mannitol et/ou le xylitol.

7. Produit médical exempt de sympathomimétiques conçu pour être utilisé selon l'une quelconque des revendications 1 à 6 sous forme de solution aqueuse.

8. Utilisation d'un produit médical exempt de sympathomimétiques selon l'une quelconque des revendications 1 à 7 pour la production d'un agent pour le traitement d'irritations des muqueuses nasales provoquées par un rhume, un rhume des foins, un nez sec et/ou une dépendance de sympathomimétiques.
